# EUROPÄISCHE PATENTSCHRIFT

(11) **EP 1 430 085 B1**
(45) Veröffentlichungstag und Bekanntmachung des Hinweises auf die Patenterteilung: **05.08.2009**
(21) Anmeldenummer: 02782679.1
(22) Anmeldetag: 16.09.2002
(51) Int. Cl.: C07K 14/705, C12N 5/06, A61K 38/17

(54) **LEUKOZYTEN-INAKTIVIERUNGS-MODUL**
LEUKOCYTE INACTIVATION MODULE
MODULE D'INACTIVATION DE LEUCOCYTES

(30) Priorität: 27.09.2001 DE 10147638
(43) Veröffentlichungstag der Anmeldung: 23.06.2004
(73) Patentinhaber: LeukoCare AG, 81379 München (DE)
(72) Erfinder: SCHOLZ ,Martin, 60590 Frankfurt (DE)
(74) Vertreter: Strehl Schübel-Hopf & Partner
(86) Internationale Anmeldenummer: PCT/DE2002/003466
(87) Internationale Veröffentlichungsnummer: WO 2003/031473

(56) Entgegenhaltungen:
- WO-A-95/18665
- WO-A-97/12632
- WO-A-97/38707
- WO-A-98/46242
- WATSON R W G ET AL: "Regulation of Fas antibody induced neutrophil apoptosis is both caspase and mitochondrial dependent" FEBS LETTERS, ELSEVIER SCIENCE PUBLISHERS, AMSTERDAM, NL, Bd. 453, Nr. 1-2, 18. Juni 1999 (1999-06-18), Seiten 67-71, XP004259804 ISSN: 0014-5793
- CINATL J JR ET AL: "Decreased neutrophil adhesion to human cytomegalovirus-infected retinal pigment epithelial cells is mediated by virus-induced up-regulation of Fas ligand independent of neutrophil apoptosis." JOURNAL OF IMMUNOLOGY (BALTIMORE, MD.: 1950) UNITED STATES 15 OCT 2000, Bd. 165, Nr. 8, 15. Oktober 2000 (2000-10-15), Seiten 4405-4413, XP002229733 ISSN: 0022-1767

## Beschreibung

### Gebiet der Erfindung

Die vorliegende Erfindung betrifft ein Leukozyten-Inaktivierungs-Modul (LIM) und ein Verfahren zum Herabsetzen der Aktivität von Leukocyten.

### Hintergrund der Erfindung

Bei verschiedenen klinischen Situationen kommt es häufig zu akut erhöhter pathologischer zellulärer Immunantwort. Beispiele dafür sind:
- Arterielle oder venöse Linie der Herz-Lungen-Maschine während herzchirurgischer Eingriffe;
- Systemic Immune Response Sydrome (SIRS) bei Polytrauma oder Sepsis (septischer Schock);
- Multiple Organ Dysfunction Syndrome (MODS) bedingt durch überschießende Immunaktivität.

Bei den oben erwähnten Eingriffen bzw. klinischen Komplikationen kommt es zur unerwünschten Aktivierung von Leukozyten. Dies führt zu schwerwiegenden pathologischen Komplikationen im Patienten. Um dies zu verhindern, sollen die aktivierten Leukozyten (insbesondere Neutrophile) aus dem Blutstrom abgefangen und sofort inaktiviert werden. Bei den derzeit erhältlichen Leukozyten-Filtern werden zwar auch vermehrt aktivierte Leukozyten aus dem Blut herausgefiltert. Allerdings produzieren und sezernieren diese noch lebenden Zellen pathogene Substanzen (Zytokine, Enzyme, Sauerstoffradikale etc.), die für die eigentliche Pathogenese verantwortlich sind. Es gibt auch Hinweise, dass die Leukozyten im Filternetz vermutlich durch den mechanischen StreB und durch den Kontakt der Zellen mit der Fremdoberfläche noch zusätzlich aktiviert werden. So wird z.B. vermehrt das von aktivierten Neutrophilen produzierte Enzym Elastase sezerniert. Elastase greift u.a. die Extrazellulärmatrix der Gefäßwand an und spaltet interendotheliale Zell-Zell Kontakte. Es kommt zur erhöhten Permeabilität der Gefäßwände, zu Ödemen, zu erhöhter Inflammation etc.

Die Apoptose ist eines der wichtigsten Regulations-Elemente des Immunsystems. Die Apoptose immunrelevanter Zellen führt dazu, dass nach erfolgter Immunantwort wie z.B. gegen mikrobielle Pathogene die Aktivität des Immunsystems wieder normalisiert wird. Auch während der Individualentwicklung muß das Immunsystem solche Immunzellen abtöten, die gegen körpereigene Strukturen oder gegen natürliche Antigene aus der Umwelt vorgehen (z.B. bei Autoimmunerkrankungen oder Allergien). T-Zellen, die über sog. Antigen-präsentierende Zellen (APZ) aktiviert werden, erhalten in der Regel mehrere Informationen. Das von den APZ aufgearbeitete Antigen wird in der Gruppe des MHC-I oder MHC-II Moleküls präsentiert. Ist die Affinität des T-Zell-Rezeptors zu dem Antigen zu schwach oder fehlen kostimulatorische Signale (z.B. über Adhäsionsmoleküle), wird die Zelle apoptotisch. Ein anderer wesentlicher Mechanismus, der zur Apoptose führt, wird über dem Fas/FasL·Weg eingeleitet. Dabei können z.B. auch entotheliale Zellen der Gefäßwand oder andere Epithelzellen FasL exprimieren, um somit das Gewebe vor eindringenden aktivierten Immunzellen zu schützen.

### Der Erfindung zugrunde liegende Aufgabe

Ziel der vorliegenden Erfindung ist es, eine Vorrichtung bereitzustellen, die geeignet ist, die Aktivität von Leukocyten zu senken und so die Sezernierung pathogener Substanzen durch die Leukocyten zu verringern. Außerdem soll ein in vitro Verfahren zur Herabsetzung der Aktivität von Leukocyten unter Einsatz einer solchen Vorrichtung bereitgestellt werden.

### Mittel zum Lösen der Aufgabe

Arbeiten der Erfinder der vorliegenden Erfindung mit Cytomegalievirus-infizierten retinalen Pigmentepithelzellen aus dem humanen Auge zeigten, daß aktivierte Neutrophile bedingt durch einen kurzen Kontakt mit FasL auf den Epithelzellen ihre Fähigkeit verloren, die Anhaftung an die re Fähigkeit verloren, die Anhaftung an die Epithelzellen aufrechtzuerhalten oder zu verstärken. Dieser überraschende Befund wird als Schutzmechanismus des Endothels und des entsprechenden Gewebes gegenüber inflammatorischer Ereignisse gewertet. Der funktionelle Verlust der Neutrophilen-EffektorMechanismen konnte innerhalb von Minuten nach Zell-Zell-Kontakt beobachtete werden und scheint zu einem großen Anteil unabhängig vom apoptotischen Signalweg innerhalb des Neutrophilen zu sein. Aufgrund dieser überraschenden Erkenntnisse ergibt sich die Nutzung beispielsweise des Fas/FasL Weges oder anderer frühen Hemm-Mechanismen der Leukozyten-Effektor-Funktionen für den experimentellen und klinischen Einsatz bei akuten überschießenden Immunreaktionen.

### Gegenstand der Erfindung

Die vorliegende Erfindung stellt ein Modul zum Herabsetzen der Aktivität von Leukocyten bereit, welches einen Bluteinström- und einen Blutausström- Stutzen, einen Träger und einen Liganden umfaßt, der an den Träger gebunden ist und zur Wechselwirkung mit einem Rezeptor von Leukocyten geeignet ist worin der Ligand das FasL₋Protein oder ein Antikörper gegen das Fas-Protein von Leukocyten ist. Außerdem stellt die vorliegende Erfindung ein in vitro Verfahren zum Herabsetzen der Aktivität von Leukocyten unter Einsatz dieses Moduls bereit.

Der Vorteil der vorliegenden Erfindung ist, daß nach Bindung der aktivierten Leukozyten im Leukocyten-Inaktivierungs-Modul (LIM) innerhalb von Minuten die schädigende Aktivität der Zellen gehemmt wird. Dies geschieht durch den Kontakt bestimmter Rezeptoren auf der Zellmembran der Leukocyten mit den entsprechenden Liganden in dem LIM. Die Liganden sind Proteine , die nach Kontakt mit dem Rezeptor auf der Zellmembran ein Signal induzieren, das wiederum die Leukocyten veranlaßt, die sekretorische Aktivität und die Immunogenität zu reduzieren. Eine Möglichkeit dies zu erreichen, ist die Induktion der Apoptose über relevante Rezeptor-Liganden-Interaktionen, wie Fas/FasL.

### Beschreibung der Erfindung

Das erfindungsgemäße Modul eignet sich zum Einbringen in den Patienten-Blutstrom über einen Shaldon-Katheter bzw. in den Kreislauf der Herz-Lungen-Maschine.

Das Modul besteht vorzugsweise aus einem KunststoffGehäuse mit z.B. 10 cm Durchmesser. Die Bluteinström- und Blutausströmstutzen sind in ihrem Durchmesser an die Schlauchverbindungen der Herz-Lungen-Maschine angepaßt. Im Innern des Moduls befindet sich ein Träger, wie eine dreidimensional gefaltete Polyestermembran mit modifizierter Oberfläche, zur Anhaftung von aktivierten Leukozyten und deren Inaktivierung und Abtötung (z.B. Apoptose-Induktion) durch Rezeptor-vermittelte Signale. Das Trägermaterial kann jegliches Material sein, das geeignet ist, Liganden zu binden. Der im vorliegenden Zusammenhang verwendete Ausdruck "Bindung" umfaß sowohl die kovalente als auch die nicht-kovalente Bindung, wie Salzbindung, hydrophobe Wechselwirkung, Affinitätsbindung, eines Liganden an den Träger. Außerdem kann der Ligand direkt oder indirekt an den Träger gebunden sein. Die indirekte Bindung umfaßt die Bindung über einen Bindungsvermittler, wie ein langkettiges Molekül zur besseren Präsentation des Liganden oder eine Zelle, die den Liganden aufweist und über eine andere Bindungswechselwirkung an den Träger haftet.

Das erfindungsgemäße LIM ist für jegliche Leukocyten geeignet, d.h. für B-Lymphocyten, T-Lymphocyten, Granulocyten, Neutrophile.

Die sonstigen Parameter des Moduls, die Blutstrom, Druck oder Rheologie bestimmen, sind wie bei herkömmlichen Leukozytenfiltern, die bereits klinisch eingesetzt werden.

### Beispiel 1:

Vertiefungen (wells) von 12-Loch-Zellkulturplatten wurden mit Polyestermembranen (40 µm Porengröße) ausgelegt und über Nacht mit verschiedenen Konzentrationen eines funktionell aktivierenden IgM-Antikörpers gegen Fas (CD95) inkubiert. Als Kontrolle dienten:
Vertiefungen ohne Membran
Vertiefungen mit Membran ohne Vorinkubation mit Antikörper Vertiefungen mit Membran mit Vorinkubation mit irrelevanten IgM-Antikörpern.

Als Testzellen wurden Fas exprimierende (Fas+) und eine Fas deletierte (Fas-; exprimiert kein Fas auf der Oberfläche) Jurkat-Zellen in einer Konzentration von 1 x 10⁶/ml den Vertiefungen für 24 h zugegeben.

Die Apoptoserate und die Nekroserate wurde mittels eines Annexin-Bindungs-Assays durchflußzytometrisch quantitativ bestimmt.

Ergebnisse: Die Fas-Jurkat-Zellen zeigten keine signifikante Erhöhung in der Annexin V-Bindungseigenschaft nach Kultivierung in den vorbehandelten Vertiefungen. Dagegen zeigte sich in Abhängigkeit von der Konzentration des IgM-Antikörpers eine deutliche Induktion der Apoptose.

| | Fas+ | Fas- |
|---|---|---|
| 0 ng | 24,81 % | 24,29 % (Kontrolliert) |
| 10 ng | 31,38 % | 27,57 % |
| 50 ng | 40,95 % | 26,20 % |
| 100 ng | 89,31 % | 29,65 % |

Bei den o.a. Kontrollen konnte keine Apoptose-Induktion festgestellt werden. Ähnliche Ergebnisse wurden mit frisch isolierten Neutrophilen erzielt.

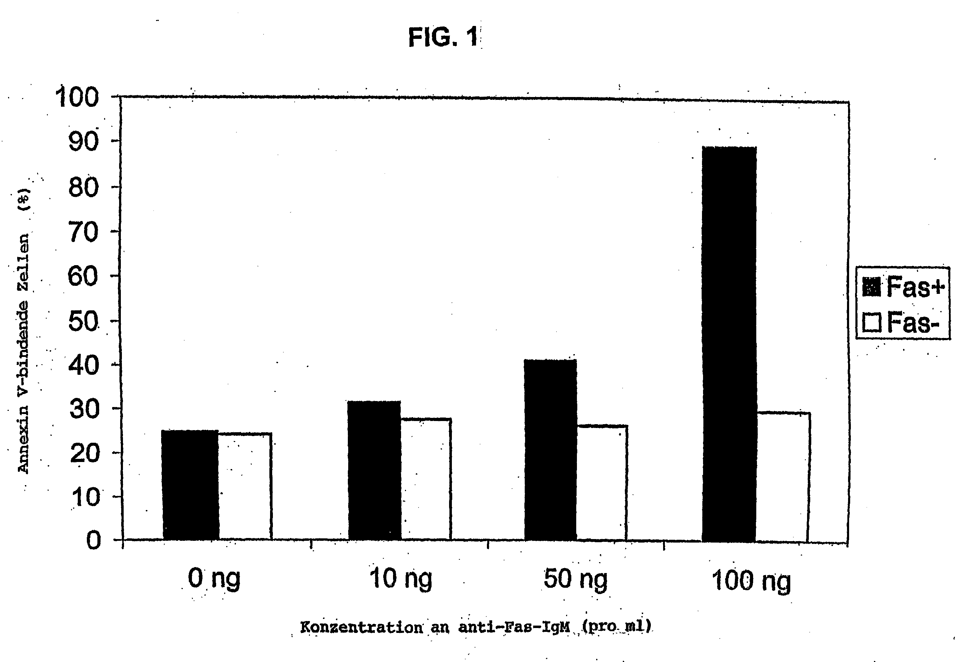

Fig. 1 zeigt die Ergebnisse des erfindungsgemäßen Beispiels.

## Patentansprüche

1. Modul zum Herabsetzen der Aktivität von Leukocyten, welches einen Bluteinström - und einen Blutausströmstutzen, einen Träger und einen Liganden umfaßt, der an den Träger gebunden ist und zur Wechselwirkung mit einem Rezeptor von Leukocyten geeignet ist, worin der Ligand das FasL-Protein oder ein Antikörper gegen das Fas-Protein von Leukocyten ist.

2. Modul nach Anspruch 1, worin der Ligand an einen Bindungsvermittler gebunden ist, der an den Träger gebunden ist.

3. Modul nach Anspruch 2, worin der Bindungsvermittler eine Zelle ist.

4. In vitro Verfahren zum Herabsetzen der Aktivität von Leukocyten durch Kontaktieren mit Liganden in einem Modul nach einem der Ansprüche 1 bis 3.

5. Verfahren nach Anspruch 4, wobei die Leukocyten nach Kontaktieren mit den Liganden weniger Zytokine und/oder Enzyme und/oder Sauerstoffradikale sezernieren als vor dem Kontaktieren.

6. Verfahren nach Anspruch 4 oder 5, wobei die Wechselwirkung des Liganden, der an eine Zelle gebunden ist, mit dem Rezeptor der Leukocyten die Aktivität, ausgedrückt als Bindungsaffinität zu der Zelle, der Leukocyten herabsetzt.

## Claims

1. A module for reducing the activity of leukocytes, which comprises a blood inlet nozzle and a blood outlet nozzle, a carrier and a ligand that is linked to the carrier and is suitable for interacting with a leukocyte receptor, wherein the ligand is the FasL protein or an antibody against the Fas protein of leukocytes.

2. The module according to claim 1, wherein the ligand is linked to a binding mediator that is linked to the carrier.

3. The module according to claim 2, wherein the binding mediator is a cell.

4. An in vitro process for reducing the activity of leukocytes by contacting with ligands in a module according to any one of claims 1 to 3.

5. The process according to claim 4, wherein the leukocytes, after contacting with the ligands, secrete cytokines and/or enzymes and/or oxygen radicals in lower amount than before contacting.

6. The process according to claim 4 or 5, wherein the interaction of the ligand linked to a cell with the leukocyte receptor reduces the activity of the leukocytes in terms of binding affinity to the cell.

## Revendications

1. Module pour diminuer l'activité de leucocytes, lequel comprend une tubulure d'entrée de sang et une tubulure de sortie de sang, un support et un ligand qui est lié au support et qui convient à l'interaction avec un récepteur de leucocytes, où le ligand est la protéine FasL ou un anticorps dirigé contre la protéine Fas de leucocytes.

2. Module selon la revendication 1, où le ligand est lié à un médiateur de liaison qui est lié au support.

3. Module selon la revendication 2, où le médiateur de liaison est une cellule.

4. Procédé in vitro de diminution de l'activité de leucocytes par mise en contact avec des ligands dans un module selon l'une des revendications 1 à 3.

5. Procédé selon la revendication 4, les leucocytes après la mise en contact avec les ligands, sécrétant moins de cytokines et/ou d'enzymes et/ou de radicaux oxygène qu'avant la mise en contact.

6. Procédé selon la revendication 4 ou 5, l'interaction du ligand lié à une cellule avec le récepteur des leucocytes diminuant l'activité, exprimée en affinité de liaison pour la cellule, des leucocytes.
